# EUROPÄISCHE PATENTSCHRIFT

(11) **EP 1 797 325 B1**
(45) Veröffentlichungstag und Bekanntmachung des Hinweises auf die Patenterteilung: **05.03.2008**
(21) Anmeldenummer: 05793032.3
(22) Anmeldetag: 27.09.2005
(51) Int. Cl.: F04B 43/12, F04B 43/10

(54) **PERISTALTISCHE PUMPE**
PERISTALTIC PUMP
POMPE PERISTALTIQUE

(30) Priorität: 01.10.2004 DE 102004048408; 05.11.2004 DE 102004053583
(43) Veröffentlichungstag der Anmeldung: 20.06.2007
(73) Patentinhaber: Universitätsklinikum Freiburg, 79102 Freiburg (DE)
(72) Erfinder: BEYERSDORF, Friedhelm, 79104 Freiburg (DE)
(74) Vertreter: Rösler, Uwe
(86) Internationale Anmeldenummer: PCT/EP2005/010429
(87) Internationale Veröffentlichungsnummer: WO 2006/037519

(56) Entgegenhaltungen:
- EP-A- 0 192 574
- EP-A- 0 478 600
- DE-A1- 3 538 718
- GB-A- 2 371 230
- US-A- 3 885 251
- US-A- 4 014 318

## Beschreibung

### Technisches Gebiet

Die Erfindung bezieht sich auf eine peristaltische Pumpe, insbesondere für einen extra- oder intrakorporalen medizinischen Einsatz.

### Stand der Technik

In Fällen auftretender Herzinsuffizienz ermöglichen unterschiedliche Therapietechniken die Herzfunktion zu unterstützen. Die meisten der bekannten diesbezüglichen Vorschläge sehen die Implantation einer künstlichen Pumpvorrichtung in einen geeigneten Abschnitt des Gefäßsystems vor. So sind axialfördernde Schraubwendelpumpen bekannt, die nach dem Wirkprinzip der archimedischen Pumpe arbeiten und eine Implantation der Pumpe in das Gefäßsystem erfordern. Zudem werfen derartige im Gefäß innen liegende, d.h. unmittelbar im Blutstrom befindliche Fördereinheiten das Problem ihres mechanischen Antriebes auf.

Ferner sind auch Pumpvorrichtungen vorgeschlagen worden, die extern um einen Gefäßabschnitt anzuordnen sind, ohne dabei Komponenten der Pumpe in das Innere des Gefäßes implantieren zu müssen. Ein Vorschlag richtete sich auf einen in- und desufflierbaren Ballonkatheter, der um die absteigende Hauptschlagader positioniert worden ist und durch taktweises In- sowie Desufflieren des Ballons wurde ein gezielter zusätzlicher Druck von Außen auf die natürliche Gefäßwand ausgeübt. Es zeigte sich jedoch, dass bereits nach mehrmaligen Deformationen des Ballonkatheters und die damit einhergehende Druckausübung auf die natürliche Gefäßwand irreversible Gefäßwandschädigungen auftraten, weswegen sich ein derartiges Vorgehen als unpraktikabel erwies.

Ein weiterführender Vorschlag zur Vermeidung der vorstehenden Gefäßwandirritationen sah im Unterschied zur Verwendung eines künstlichen Ballonkatheters die Umwickelung eines Bereiches der Hauptschlagader mit einem Muskel vor, der aus dem großen Rückenmuskel entnommen wurde und mit Hilfe eines künstlichen Schrittmachers zur Ausübung gezielter Kontraktionen aktiviert wurde. Zwar traten in diesem Fall die vorstehend erwähnten Gewebewandirritationen nicht oder zumindest nicht in einem auffallenden Maße auf, doch erwies sich der Einsatz von transplantiertem Muskelmaterial als wenig langzeitstabil, weswegen auch dieser Lösungsvorschlag wenig Aussicht auf Erfolg eröffnete.

Aus der DE 690 33 350 T2 sind Maßnahmen zur Verbesserung der Blutströmung entnehmbar, die sich durch zwei in der Druckschrift beschriebene alternative Ausführungsbeispiele realisieren lassen. So nutzt eine Alternative eine ringförmige Hülse, die eine Druckkraft entlang ihrer Länge befördert, um den Blutfluss in dem Gefäß zu unterstützen. Ein diesbezügliches Ausführungsbeispiel einer derartigen peristaltischen Pumpe umfasst einen Mantel, eine Hülse oder einen Bund, der bzw. die im Gebrauch die sog. Pfortader umgibt. Das zweite Ausführungsbeispiel weist eine Mehrzahl von aufblasbaren Elementen auf, die so angeordnet sind, dass sie auf dem Gefäß liegen oder das Gefäß zwischen ihnen angeordnet ist. So bedarf es bei beiden Ausführungsformen der bekannten peristaltischen Pumpe dreier längs der Manschette angeordnete, jeweils separat voneinander in- bzw. desufflierbare Elemente bzw. Segmente, die für eine unidirektionale Fluidströmungsrichtung das Blutgefäß taktweise von außen zu deformieren haben und ferner das Blutgefäß zur nahezu fluiddichten Abdichtung lokal förmlich einquetschen, um eine entsprechende Schleusenwirkung zu erzielen. Somit sind mit den beschriebenen Maßnahmen folgende Nachteile verbunden: Aufgrund der längs eines Blutgefäßes anzuordnenden und separat in- sowie dezusufflierenden Luftvolumina bedarf es einer aufwendigen Steuer- und Regelungstechnik, um die einzelnen Dillatierelemente aufeinander abgestimmt taktweise betreiben zu können. Insbesondere gilt es für den Insufflationsvorgang darauf zu achten, dass keine Überbeanspruchung am Gewebe auftritt. Dies hätte irreversible Gewebeschädigungen zur Folge und eine damit verbundene Lebensgefahr.

### Darstellung der Erfindung

Der Erfindung liegt daher die Aufgabe zugrunde, eine Pumpvorrichtung anzugeben, mit dem hauptsächlichen Ziel einer intrakorporalen Anwendung zumindest zur Unterstützung der Herzfunktion, um den Blutfluss durch ein Gefäßsystem künstlich zu unterstützen bzw. aufrecht zu erhalten. So soll die Pumpe einfach und zuverlässig ansteuerbar, einfach und zuverlässig implantierbar sowie gewebeschonend für jenen Bereich des Blutgefäßes sein, längs dem die peristaltische Pumpenanordnung den Blutstrom zu unterstützen hat.

Die Lösung der der Erfindung zugrunde liegenden Aufgabe ist im Anspruch 1 angegeben. Den Erfindungsgedanken vorteilhaft weiterbildende Merkmale sind Gegenstand der Unteransprüche sowie unter Bezugnahme auf die Ausführungsbeispiele beschrieben.

Die erfindungsgemäße peristaltische Pumpe, die für einen extra- aber insbesondere für einen intrakorporalen medizinischen Einsatz geeignet ist, weist einen, über eine flexible Kanalwand verfügenden Durchströmungskörper auf, dessen flexible Kanalwand zumindest abschnittsweise einen Durchströmungskanal umschließt, der ein stromauf- und ein stromabwärtiges Kanalende aufweist, sowie mit wenigstens einem zur Induzierung einer Fluidströmung längs des Durchströmungskanals mit der flexiblen Kanalwand in Wirkverbindung stehendes Mittel vorsieht, durch das die flexible Kanalwand vorgebbar deformierbar ist. Am stromauf- und stromabwärtigen Kanalende sind mittel- oder unmittelbar jeweils ein in Art einer künstlichen Herzklappe ausgebildetes Schleusenelement derart vorgesehen, dass ein sich innerhalb des Durchströmungskanals befindliches Fluid ausschließlich in eine durch den Durchströmungskanal und die Schleusenelemente vorgebbare Strömungsrichtung auszubreiten vermag. Das Mittel ist in Art eines toroidalen Ballons ausgebildet, der um den Durchströmungskörper angebracht, mit der flexiblen Kanalwand verbunden und über wenigstens eine Leitung mit einem Fluid, vorzugsweise mit einem Gas, in- und desufflierbar ist.

Der wesentliche Vorteil der Pumpe ist zum einen darin zu sehen, dass keine Komponenten der Pumpe innerhalb des zu fördernden Fluids längs des Strömungskanals vorgesehen sind. Ein Fluidstrom wird längs des Strömungskanals dadurch induziert, indem eine elastische, den Strömungskanal eingrenzende Kanalwand durch äußere Kraft- bzw. Druckeinwirkung auf die Kanalwand deformiert wird, wodurch das Fluid innerhalb des Strömungskanals verdrängt wird.

Im Unterschied zu den bisher bekannten Lösungsansätzen besteht sowohl der Durchströmungskörper als auch das mit der flexiblen Kanalwand des Durchströmungskörpers in Wirkverbindung stehende Mittel zur gezielten Deformation der flexiblen Kanalwand, aus künstlichen, vorzugsweise gummi- oder kunststoffartigen Materialien, die hohen mechanischen Beanspruchungen standhalten können und somit für einen dauerhaften Einsatz geeignet sind. Im Falle der Implantation der erfindungsgemäß ausgestalteten peristaltischen Pumpe im Bereich eines blutführenden Gefäßes, ist der Durchströmungskörper längs der natürlichen Gefäßbahn zu integrieren. Dies kann realisiert werden, indem das stromauf- und stromabwärtige Ende des Durchströmungskörpers jeweils mit den offenen Enden eines durchtrennten Gefäßkanals verbunden, beispielsweise vernäht werden. Der Durchströmungskörper ist somit in Art eines Distanzstückes längs der natürlichen Gefäßbahn integriert. An der Außenseite der flexibel ausgebildeten Kanalwand ist der Durchströmungskörper mit dem die Kanalwand deformierenden Mittel, vorzugsweise in Art eines in- und desufflierbaren Volumenelementes, vorzugsweise in Art eines Ballons, verbunden.

Die Schleusenelemente sind jeweils in Art einer Einwegschleuse für eine einheitliche Durchströmungsrichtung des Durchströmungskörpers ausgebildet. Zwischen den beiden Schleusenelementen wird zur gezielten Deformation der Kanalwand vorzugsweise ein dilatierbarer Ringballon angebracht, der mit der äußeren Kanalwand fest verbunden ist. Wird der Ballon insuffliert, beispielsweise mit Helium, so wird der Strömungskanal zusammengequetscht, die zwischen beiden Schleusenelementen vorhandene Fluidmenge wird infolgedessen in eine Richtung durch die sich öffnende Schleuse ausgestoßen. Wird der Ringballon hingegen desuffliert, so wird die fest mit dem Ringballon verbundene Kanalwand in ihren ursprünglichen Zustand zurückgezogen, wodurch sich das Kanalvolumen zwischen beiden Schleusenelementen vergrößert. Im Zuge der Volumenvergrößerung öffnet die gegenüberliegende Schleuse und aufgrund eines sich einstellenden Unterdruckes wird der Kanalbereich wieder mit Fluid gefüllt. Der Vorgang des Fluidausstoßes sowie der Befüllung des Kanalvolumens erfolgt taktweise, vorzugsweise in Anpassung an den natürlichen Puls des menschlichen Blutkreislaufes.

Untersuchungen am menschlichen Gefäßsystem haben ergeben, dass Patienten mit zu hohem Blutdruck sowie einem zu großen Pulshöhenunterschied zwischen dem systolischen und diastolischen Blutdruckwert einer erhöhten Gefahr der Arterienverkalkung unterliegen. Untersuchungen an Patienten mit normalen Blutdruckwerten und insbesondere niedrigen Druckunterschieden zwischen systolischem und diestolischem Blutdruckwert zeigten eine weit geringere Arterienverkalkung, ja sogar in Fällen eines weitgehend mit geringen Pulswellen beaufschlagten Blutstromes eine Rückbildung vorhandener Arterienverkalkungen. Dies lässt den Schluss zu, dass eine gleichmäßige axial gerichtete laminare Blutströmung äußerst positive Auswirkungen auf reduziertere Ablagerungen von Blutbestandteilen im Inneren der Gefäßwände hat. Patienten mit einem hohen Blutfettanteil und dadurch bedingten erhöhten Ablagerungserscheinungen an den Arterienwänden, vorzugsweise LDL-Ablagerungen in den Arterienwänden, könnte effektiv dadurch geholfen werden, indem der natürliche Blutfluss auf ein normales Druckniveau eingestellt wird, ohne dabei einer durch den Herzschlag bedingten überhöhten Pulswelle zu unterliegen. Zielführend hierfür ist der Einsatz der lösungsgemäßen peristaltischen Pumpe, die im natürlichen Blutkreislauf an einer Stelle, vorzugsweise längs der Brustschlagader implementiert wird. Die peristaltische Pumpe ist durch entsprechende Betätigung des in- und desufflierbaren Mittels derart zu betreiben, dass die durch die peristaltische Pumpe erzeugte Blutströmung eine weitgehend axial gerichtete laminare Strömungscharakteristik mit einer weitgehend vernachlässigbaren Pulswelle aufweist. In einer bevorzugt ausgebildeten Ausführungsvariante sieht die peristaltische Pumpe darüber hinaus im Inneren ein Mittel zum Ausfiltern bestimmter Blutstoffbestandteile vor, wie beispielsweise zum Ausfiltern von Blutfetten, wie LDL. Derartige Filtermittel können in Form entsprechend konfektionierter im Bereich des Druchströmungskanals angeordneter Diaphragmen oder durch Verabreichen von an der peristaltischen Pumpe vorgesehener Antikörper, die in Reservoir-Volumina vorgesehen sind, ausgeführt werden.

Die lösungsgemäße peristaltische Pumpe vermag somit einerseits zur Blutdruckregulation als auch zur Pulswellenregulation beitragen. Insbesondere ist es vorteilhaft die Aktivität der peristaltischen Pumpe unter Zugrundelegung externer Mess- oder Regelgrößen getriggert zu betreiben. Als geeignete Messgröße dient beispielsweise der an bestimmten Gefäßbereichen vorherrschende Blutdruck oder Blutfluss, der messtechnisch in geeigneter Weise erfassbar ist. Ebenso ist der EKG-Wert als die Herzfunktion beschreibende Vitalgröße ein wichtiger Parameter, an dem gemessen die Aktivität der peristaltischen Pumpe einstellbar ist. Der Abgleich der Pumpenaktivität an einen vorgebbaren Sollwert unter Berücksichtigung eines in situ erfassten Messwertes ermöglicht somit grundsätzlich den Einsatz der peristaltischen Pumpe in einem geschlossenen oder offenen Regelkreis.

Eine weitere, alternative intrakorporale Anwendungsmöglichkeit der lösungsgemäß ausgebildeten peristaltischen Pumpe sieht die Integration der peristaltischen Pumpe im Bereich der Beckenschlagader vor, um in diesem Gefäßbereich den Blutdruck zusätzlich zu unterstützen. So verhilft die peristaltische Pumpe das Blut, beispielsweise unter gleichzeitigem Zusatz von Wachstumsfaktoren, wie beispielsweise VEGF, in das natürlich vorhandene Adernsystem mit entsprechend erhöhtem Druck zu fördern, wodurch die natürliche Adernausbildung unterstützt wird.

Ein weiteres besonderes interessantes Ausführungsbeispiel geht von einem vollkommen autark arbeitenden, den natürlichen Blutstrom unterstützenden System aus, das keiner externen Energieversorgung bedarf. Hierzu ist es erforderlich die zur gezielten Förderung des Fluidstromes zur taktweisen Befüllung und Entleerung des um den Durchströmungskörper angebrachten toroidalen Ballons notwendige Energie aus körpereigenen Energieressourcen zu entnehmen und somit auf jegliche externe Energiequelle, wie bspw. einen externen De- und Insufflator zu verzichten. Hierdurch kann die Patientenbeeinträchtigung auf ein unbeutendes Maß reduziert werden, wodurch die Akzeptanz einer derartigen Vorrichtung erheblich verbessert werden kann. Selbst im Falle eines nicht vollständigen Verzichtes auf eine externe Energiezuführung, zumal die körpereigene Energieversorgung nicht bei jedem Patienten vorausgesetzt werden kann, insbesondere bei jenen Patienten mit reduzierter oder eingeschränkter Atemaktivität, trägt eine intrakorporal vorgesehene, an die Atembewegung gekoppelte Pumpeneinrichtung für das den toroidalen Ballon zu befüllende Fluid dazu bei, den erforderlichen externen Energieeintrag zu reduzieren, wodurch leistungsärmere und somit weniger optisch als auch akustisch beeinträchtigend in Erscheinung tretende Insufflatoren eingesetzt werden können.

Die an die Atembewegung gekoppelte implantierbare Pumpeneinrichtung sieht wenigstens ein Reservoirvolumen vor, das von einer elastischen Hülle umschlossen und mit dem Brustkorb und/oder dem Zwerchfell derart verbunden ist, dass die Hülle in Abhängigkeit der Atembewegung gedehnt oder komprimiert wird, wodurch das innerhalb des Reservoirvolumens befindliche Fluid, vorzugsweise in Form von Gas, aus dem Reservoirvolumen verdrängt oder in das Reservoirvolumen eingesaugt wird. Unter Nutzung der durch die Atembewegung hervorgerufene Brustkorb- sowie Zwerchfell-Bewegung eröffnet sich somit grundsätzlich die Möglichkeit eines autark arbeitenden peristaltischen Pumpensystems. Denkbar wäre auch eine Kombination der Pumpeneinrichtung mit einer Energiewandlervorrichtung, mit der es möglich ist körpereigener Energieressourcen zu nutzen und die gewonnene Energie in elektrische Energie umzuwandeln, die in einer implantierbaren Batterieeinheit zwischengepuffert und bei Bedarf abgerufen werden kann.

Neben der vorstehend erläuterten Nutzung der Atmungsbedingten Brustkorb- und/oder Zwerchfellbewegung bestehen weitere Möglichkeiten zur Nutzung körpereigener Energieressourcen, wie bspw. die Nutzung der Herzmuskelaktivität oder des Bewegungsapparates des Menschen bspw. die muskelunterstützte Bewegungsenergie der Extremitäten.

Neben den intrakorporalen Anwendungsmöglichkeiten kann die lösungsgemäße peristaltische Pumpe auch für extrakorporale Zwecke eingesetzt werden, wie beispielsweise im Rahmen einer Herz-Lungen-Maschine, die bis anhin mit so genannten Rollenpumpen betrieben wird, durch die der flexibel ausgebildete Förderschlauch nicht nur bezüglich seines Schlauchdurchmessers, sondern auch im Wege des Überstreifens der Rollenelemente in Längsrichtung entsprechenden Dehnungen und Stauchungen unterliegt, wodurch der Schlauch erheblich mechanisch beansprucht wird. Die lösungsgemäß ausgebildete peristaltische Pumpe gewährleistet systembedingt eine weit geringere Materialbeanspruchung des Förderschlauches und sichert somit eine längere Einsatzbereitschaft.

### Kurze Beschreibung der Erfindung

Die Erfindung wird nachstehend unter Beschränkung des allgemeinen Erfindungsgedankens anhand von Ausführungsbeispielen unter Bezugnahme auf die Zeichnungen exemplarisch beschrieben. Es zeigen:
- Fig. 1: schematisierte Darstellung einer peristaltischen Pumpe mit zwei Einwegschleusen,
- Fig. 2a, b: schematisierte Sequenzbilddarstellungen für den Ausstoß und Ansaugtakt der Pumpenanordnung und
- Fig. 3: schematisierte Detaildarstellung eines Querschnittes durch ein in- und desufflierbares Mittel.

### Wege zur Ausführung der Erfindung, gewerbliche Verwendbarkeit

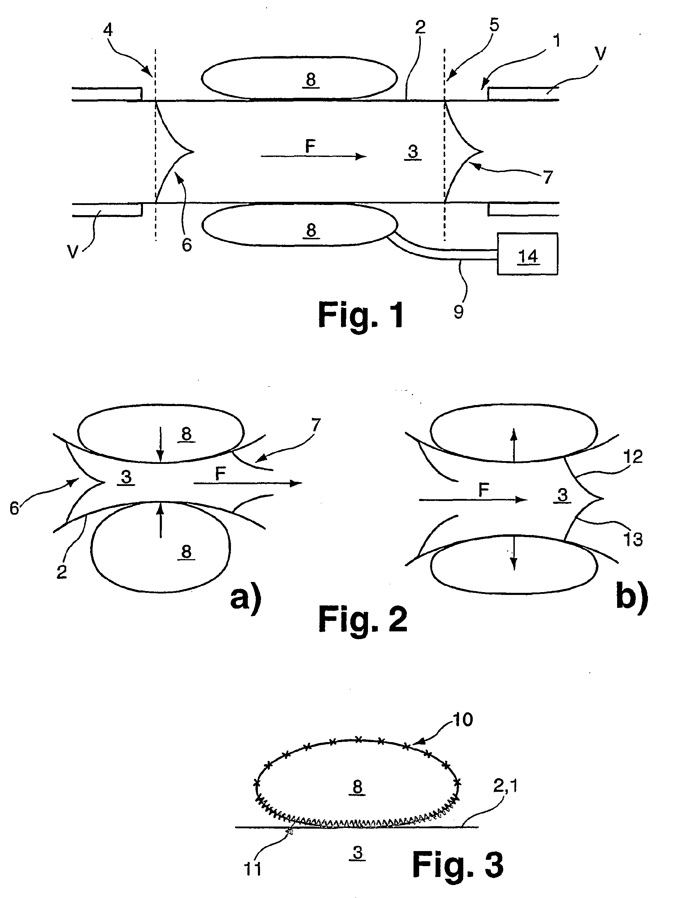

In Fig. 1 ist eine stark schematisierte Darstellung einer peristaltischen Pumpe dargestellt, die einen Durchströmungskörper 1 vorsieht, der eine flexible Kanalwand 2 aufweist, die einen inneren Durchströmungskanal 3 umschließt. Der Durchströmungskörper 1 weist ein stromaufwärtiges Kanalende 4 sowie ein stromabwärtiges Kanalende 5 auf. Am stromaufwärtigen Kanalende 4 sowie auch am stromabwärtigen Kanalende 5 ist jeweils ein Schleusenelement 6 bzw. 7 vorgesehen, das in Art einer Einwegschleuse ausgebildet ist, d.h. das jeweilige Schleusenelement 6, 7 ermöglicht jeweils einen Fluiddurchtritt in die gleiche Strömungsrichtung (im dargestellten Bildbeispiel nach rechts gemäß der Pfeildarstellung F). An der Außenwand der flexibel ausgebildeten Kanalwand 2 ist ein in- und desufflierbares Mittel 8 vorgesehen, das über eine Leitung mit einem In- sowie Desufflator 14 verbunden ist. Das Mittel 8 ist in Art eines toroidalen Ballons ausgebildet und wird über den In- und Desufflator 14 mit einem Gas, vorzugsweise Helium, gefüllt bzw. von diesem entleert.

Im Falle eines intrakorporalen Einsatzes der in Fig. 1 dargestellten peristaltischen Pumpe ist das stromaufwärtige Ende 4 sowie das stromabwärtige Ende 5 jeweils fluiddicht an eine natürliche Gefäßwand V angebracht, die beispielsweise über eine Nahtverbindung mit dem Durchströmungskörper jeweils beidseitig verbunden ist. Auf diese Weise kann je nach Betriebsweise des in- und desufflierbaren Mittels 8 eine kontinuierliche oder gepulste Blutströmung durch die peristaltische Pumpe gewährleistet werden.

In Fig. 2a und b ist der Ausstoß- bzw. Einsaugvorgang in bzw. aus der peristaltischen Pumpe dargestellt. In Fig. 2a sei angenommen, dass das innere Volumen des Durchströmungskanals 3 vollständig mit einem Fluid F gefüllt ist. Durch Insufflation des Mittels 8 dehnt sich dieses aus und drückt die flexible Kanalwand 2 von allen Seiten zusammen. Durch die im Inneren stattfindende Volumenverkleinerung wird das im Inneren des Durchströmungskanals 3 befindliche Fluid durch das Schleusenelement 7 in der Bilddarstellung gemäß Fig. 2a nach rechts durch Verdrängung ausgestoßen. Da sich das Schleusenelement 6 nicht entgegen der in Fig. 2a dargestellten Durchströmungsrichtung öffnet und in Art einer Einwegschleuse wirkt, wird jeglicher Rückfluß vermieden. Nach Ausstoß des nahezu gesamten im Durchströmungskanal 3 befindlichen Fluids F durch vollständige Insufflation des Mittels 8 wird das im Inneren des Mittels 8 befindliche Gas schnellstmöglich, beispielsweise innerhalb von Zehntel Sekunden abgesaugt, wodurch sich das Mittel 8 ringförmig nach außen zurückzieht, gemäß Bilddarstellung in Fig. 2b. Hierdurch wird im Innenraum des Durchströmungskanals 3 ein Unterdruck erzeugt, wodurch das Schleusenelement 7 durch Aneinanderliegen der Schleusenklappen 12, 13 schließt und das Schleusenelement 6 durch Nachströmen eines Fluids in das Innere des Durchströmungskanals 3 öffnet. Durch taktweises Wiederholen des insufflierten bzw. desufflierten Zustandes gemäß der Bilddarstellung in Fig. 2a und b wird eine Fluidströmung F in einer durch die Durchlassrichtung der Schleusenelemente 6, 7 vorgegebenen Strömungsrichtung druckbeaufschlagt generiert werden.

Um den Insufflations- und Desufflationsvorgang in kürzester Zeit ausüben zu können, ist der aus elastischem Material gefertigte toroidale Ballon 8 mit zwei unterschiedlich elastisch ausgebildeten Ballonhälften 10, 11 ausgebildet (siehe Figur 3 , die einen Querschnitt durch das Mittel 8 zeigt). So ist die dem Durchströmungskörper 1 zugewandte Ballonoberfläche 11 elastischer ausgebildet, als die von dem Durchströmungskörper 2 abgewandte Ballonoberfläche 10. Durch die weniger elastisch ausgebildete Ballonoberfläche 10 wird insbesondere der Desufflationsvorgang gemäß Bilddarstellung Fig. 2a verbessert, wodurch die zusammengedrückten flexiblen Kanalwandabschnitte 2 durch die ringförmig vorgegebene Eigenstabilität der Ballonoberfläche 10 radial nach außen gezogen werden.

Zur unbedenklichen Implantation der peristaltischen Pumpe in einen tierischen oder menschlichen Körper sind alle Komponenten der peristaltischen Pumpe aus bioverträglichem Material, vorzugsweise bioverträglichem Kunststoffmaterial ausgeführt.

### BEZUGSZEICHENLISTE

- 1: Durchströmungskörper
- 2: Flexible Kanalwand
- 3: Durchströmungskanal
- 4: Stromaufwärtiges Kanalende
- 5: Stromabwärtiges Kanalende
- 6, 7: Schleusenelement
- 8: In- und desufflierbares Mittel
- 9: Verbindungsleitung
- 10, 11: Ballonoberfläche
- 12, 13: Klappenelemente
- 14: In- und Desufflator

## Patentansprüche

1. Peristaltische Pumpe, insbesondere für einen extra- oder intrakorporalen medizinischen Einsatz mit einem über eine flexible Kanalwand (2) verfügenden Durchströmungskörper (1), dessen flexible Kanalwand (2) zumindest abschnittsweise einen Durchströmungskanal (3) umschließt, der ein stromauf- und ein stromabwärtiges Kanalende (4, 5) aufweist, sowie mit wenigstens einem zur Induzierung einer Fluidströmung (F) längs des Durchströmungskanals (3) mit der flexiblen Kanalwand (2) in Wirkverbindung stehenden Mittel (8), durch das die flexible Kanalwand (2) vorgebbar deformierbar ist,
**dadurch gekennzeichnet, dass** an dem stromauf- und stromabwärtigen Kanalende (4, 5) mittel- oder unmittelbar jeweils ein in Art einer künstlichen Herzklappe ausgebildetes Schleusenelement (6, 7) derart vorgesehen sind, dass ein sich innerhalb des Durchströmungskanals (3) befindliches Fluid ausschließlich in eine durch den Durchströmungskanal (3) und die Schleusenelemente (6, 7) vorgebbare Strömungsrichtung auszubreiten vermag, und
dass das Mittel (8) in Art eines toroidalen Ballons ausgebildet ist, der um den Durchströmungskörper (1) angebracht, mit der flexiblen Kanalwand (2) verbunden und über wenigstens eine Leitung (9) mit einem Fluid, vorzugsweise mit einem Gas, in- und desufflierbar ist.

2. Peristaltische Pumpe nach Anspruch 1,
**dadurch gekennzeichnet, dass** der toroidale Ballon eine von dem Druchströmungskörper (1) zugewandte Ballonoberfläche (11) aufweist, die elastisch ausgebildet ist und eine von dem Durchströmungskörper (1) abgewandte Ballonoberfläche (10) aufweist, die weniger elastisch ausgebildet ist als die dem Durchströmunsgkörper zugewandte Ballonoberfläche (11).

3. Peristaltische Pumpe nach Anspruch 1 oder 2,
**dadurch gekennzeichnet, dass** der toroidale Ballon eine aus bioverträglichem Material bestehende einstückige Ballonwand aufweist.

4. Peristaltische Pumpe nach einem der Ansprüche 1 bis 3,
**dadurch gekennzeichnet, dass** der toroidale Ballon als Ballonkatheter ausgebildet ist.

5. Peristaltische Pumpe nach einem der Ansprüche 1 bis 4,
**dadurch gekennzeichnet, dass** der Durchströmungskörper (1) sowie auch die Schleusenelemente (6, 7) aus bioverträglichem Material bestehen, das für eine intrakorporale Implantation geeignet ist.

6. Peristaltische Pumpe nach einem der Ansprüche 1 bis 5,
**dadurch gekennzeichnet, dass** das Schleusenelement wenigstens zwei zumindest teilelastische Klappenelemente (12, 13) aufweist, die inwandig längs des Durchströmungskanals (3) angebracht sind, sich gegenseitig fluiddicht überlappen und im sich überlappenden Zustand den gesamten Querschnitt des Durchströmungskanals (3) abdecken.

7. Peristaltische Pumpe nach einem der Ansprüche 1 bis 6,
**dadurch gekennzeichnet, dass** das in- und desufflierbare Mittel (8) mit einem In- bzw. Desufflator (14) verbunden ist, der das Mittel (8) innerhalb weniger Zehntelsekunden mit einem Fluid, vorzugsweise Gas, wie Helium, vollständig zu insufflieren und desufflieren vermag.

8. Peristaltische Pumpe nach Anspruch 7,
**dadurch gekennzeichnet, dass** der In- bzw. Desufflator (14) in Abhängigkeit einer Mess- oder Regelgröße gesteuert oder geregelt betreibbar ist.

9. Peristaltische Pumpe nach Anspruch 8,
**dadurch gekennzeichnet, dass** die Messgröße ein Blutdruckwert, ein Blutflusswert und/oder ein EKG-Wert ist.

10. Peristaltische Pumpe nach einem der Ansprüche1 bis 9,
**dadurch gekennzeichnet, dass** in dem Durchströmungskörper ein Mittel zum Ausfiltern wenigstens eines Blutbestandteil, vorzugsweise zur Filtration von Blutfetten, vorgesehen ist.

11. Peristaltische Pumpe nach einem der Ansprüche 1 bis 10,
**dadurch gekennzeichnet, dass** das in- und desufflierbare Mittel (8) mit einem In- bzw. Desufflator (14) verbunden ist, der das Mittel (8) taktweise derart mit einem Fluid, vorzugsweise Gas, wie Helium, zu insufflieren und desufflieren vermag, dass die Induzierung einer Fluidströmung (F) längs des Durchströmungskanals (3) mit einer weitgehend geringen Drucküberhöhung erfolgt und sich vorzugsweise eine axiale laminare Fluidströmung, d.h. ohne Druckwellenüberhöhung, innerhalb des Durchströmungskörpers ausbildet.

12. Peristaltische Pumpe nach einem der Ansprüche 1 bis 11,
**dadurch gekennzeichnet, dass** das in- und desufflierbare Mittel (8) mit einer inkorporal eingebrachten Pumpeneinrichtung verbunden ist, die das Mittel (8) taktweise derart mit einem Fluid, vorzugsweise Gas, wie Helium, zumindest teilweise zu insufflieren und desufflieren vermag, dass die Induzierung einer Fluidströmung (F) längs des Durchströmungskanals (3) mit einer weitgehend geringen Drucküberhöhung erfolgt und sich vorzugsweise eine axiale laminare Fluidströmung, d.h. ohne Druckwellenüberhöhung, innerhalb des Durchströmungskörpers ausbildet, und
dass die Pumpeneinrichtung eine durch die Atembewegungen des Brustkorbes und/oder des Zwerchfelles antreibbare Pumpenaktorik vorsieht.

13. Peristaltische Pumpe nach Anspruch 12,
**dadurch gekennzeichnet, dass** die Pumpenaktorik wenigstens ein mit dem Brustkorb und/oder dem Zwerchfell verbundenes Reservoirvolumen vorsieht, das von einer elastischen Hülle umschlossen und durch die Atembewegung deformierbar ist, und dass innerhalb des Reservoirvolumens wenigstens ein Teil des Fluids vorgesehen ist, das initial durch die Atembewegung aus dem Reservoirvolumen zur Insufflation des Mittels (8) verdrängt oder in das Reservoirvolumen zur Desufflation des Mittels (8) gesaugt wird.

## Claims

1. A peristaltic pump, particularly for extra- or intracorporal medical use, with a through-flow member (1) that is provided with a flexible duct wall (2), its flexible duct wall (2) enclosing at least partially a through-flow duct (3) which has an upstream and a downstream duct end (4, 5), and with at least one means (8)in operative connection with the flexible duct wall (2) to induce a fluid flow (F) along the through-flow duct (3), by which means (8) the flexible duct wall (2)is able to be deformed in a given manner,
**characterized in that**
at the upstream and downstream duct end (4, 5) a lock element (6, 7), formed in the manner of an artificial heart valve, is provided respectively indirectly or directly in such a way that a fluid situated inside the through-flow duct (3) is able to expand exclusively in a direction of flow which can be predefined by the through-flow duct (3) and the lock elements (6, 7), and
that the means (8)is formed in the manner of a toroidal balloon which is arranged around the through-flow member (1), is connected with the flexible duct wall (2) and is able to be insufflated and desufflated via at least one line (9) with a fluid, preferably with a gas.

2. The peristaltic pump according to Claim 1,
**characterized in that** the toroidal balloon has a balloon surface (11) facing the through-flow member (1), which is formed so as to be elastic and has a balloon surface (10) facing away from the through-flow member (1) which is formed so as to be less elastic than the balloon surface (11) facing the through-flow member.

3. The peristaltic pump according to Claim 1 or 2,
**characterized in that** the toroidal balloon has a balloon wall, in a single piece, consisting of bio-compatible material.

4. The peristaltic pump according to any of Claims 1 to 3,
**characterized in that** the toroidal balloon is formed as a balloon catheter.

5. The peristaltic pump according to any of Claims 1 to 4,
**characterized in that** the through-flow body (1) and also the lock elements (6, 7) consist of bio-compatible material which is suitable for intracorporal implantation.

6. The peristaltic pump according to any of Claims 1 to 6,
**characterized in that** the lock element has at least two at least partially elastic valve elements (12, 13) which are arranged on the inner wall along the through-flow duct (3), overlap each other reciprocally in a fluid-tight manner and in the overlapping state cover the entire cross-section of the through-flow duct (3).

7. The peristaltic pump according to any of Claims 1 to 6,
**characterized in that** the insufflatable and desufflatable means (8) are connected with an insufflator and desufflator (14) which is able to completely unsufflate and desufflate the means (8) within a few tenths of a second with a fluid, preferably gas, such as helium.

8. The peristaltic pump according to Claim 7,
**characterized in that** the in- and desufflator (14) is able to be operated in a controlled or regulated manner as a function of a measurement or regulating amount.

9. The peristaltic pump according to Claim 8,
**characterized in that** the measurement amount is a blood pressure value, a blood flow value and/or an ECG value.

10. The peristaltic pump according to any of Claims 1 to 9,
**characterized in that** in the through-flow member, a means is provided for filtering out at least one blood component, preferably for the filtration of blood fats.

11. The peristaltic pump according to any of Claims 1 to 10,
**characterized in that** the in- and desufflatable means (8) is connected with an in- and desufflator (14) which is able to insufflate and desufflate the means (8) in a synchronized manner with a fluid, preferably gas, such as helium, in such a way that the inducing of a fluid flow (F) takes place along the through-flow duct (3) with a substantially small pressure increase and preferably an axial laminar fluid flow occurs, i.e. without a pressure wave increase, inside the through-flow member.

12. The peristaltic pump according to any of Claims 1 to 11,
**characterized in that** the in- and desufflatable means (8) is connected with a pump device which is introduced incorporally and which is able to at least partially insufflate and desufflate the means (8) in a synchronized manner with a fluid, preferably gas, such as helium, in such a way that the inducing of a fluid flow (F) along the through-flow duct (3) takes place with a substantially small pressure increase and preferably an axial laminar fluid flow occurs, i.e. without a pressure wave increase, inside the through-flow member,
and
that the pump device provides a pump actuator arrangement which is able to be driven by the respiratory movements of the thorax and/or of the diaphragm.

13. The peristaltic pump according to Claim 12,
**characterized in that**
the pump actuator arrangement provides at least one reservoir volume connected with the thorax and/or the diaphragm, which is surrounded by an elastic casing and is deformable by the respiratory movement, and that inside the reservoir volume at least a portion of the fluid is provided which initially through the respiratory movement is displaced from the reservoir volume for the insufflation of the means (8) or is drawn into the reservoir volume for the desufflation of the means (8).

## Revendications

1. Pompe péristaltique, prévue notamment pour une utilisation médicale extracorporelle ou intracorporelle, avec un corps de passage (1) disposant d'une paroi de conduit flexible (2) dont la paroi de conduit flexible (2) entoure au moins partiellement un conduit de passage (3), comportant une extrémité de conduit en amont et en aval (4, 5) ainsi qu'avec au moins un moyen (8) pour induire un écoulement de fluide (F) le long du conduit de passage (3), qui est en interaction avec la paroi de conduit flexible (2), par lequel la paroi de conduit flexible (2) est déformable de façon prédéfinissable,
**caractérisé en ce que** sur l'extrémité de conduit en amont et en aval (4, 5), il est prévu directement ou indirectement chaque fois un élément d'éclusage (6, 7) conçu à la manière d'une valvule cardiaque artificielle, de sorte qu'un fluide se trouvant à l'intérieur du conduit de passage (3) soit susceptible de se propager dans une direction d'écoulement prédéfinissable par le conduit de passage (3) et par les éléments d'éclusage (6, 7),
et **en ce que** le moyen (8) est conçu à la manière d'un ballonnet toroïdal, qui est monté autour du corps de passage (1), relié avec la paroi de conduit flexible (2) dans lequel par l'intermédiaire d'au moins une conduite (9) on peut lui insuffler un fluide, de préférence du gaz ou l'aspirer.

2. Pompe péristaltique selon la revendication 1,
**caractérisée en ce que** le ballonnet toroïdal comporte une surface de ballonnet (11) dirigée vers le corps de passage (1), qui est conçue en version élastique et une surface de ballonnet (10) opposée au corps de passage (1), qui est conçue en version moins élastique que la surface de ballonnet (11) dirigée vers le corps de passage.

3. Pompe péristaltique selon la revendication 1 ou 2,
**caractérisée en ce que** le ballonnet toroïdal comporte une paroi de ballonnet consistant dans une matière biocompatible.

4. Pompe péristaltique selon l'une quelconque des revendications 1 à 3,
**caractérisé en ce que** le ballonnet toroïdal est conçu sous la forme d'un cathéter à ballonnet.

5. Pompe péristaltique selon l'une quelconque des revendications 1 à 4,
**caractérisée en ce que** le corps de passage (1), tout comme également les éléments d'éclusage (6, 7) consistent dans une matière biocompatible, qui est adaptée pour une implantation intracorporelle.

6. Pompe péristaltique selon l'une quelconque des revendications 1 à 5,
**caractérisée en ce que** l'élément d'éclusage comporte deux éléments valvulaires (12, 13) au moins partiellement élastiques, qui sont montés à l'intérieur de la paroi, le long du conduit de passage (3), qui se chevauchent mutuellement de façon étanche au fluide et qui en position de chevauchement recouvrent l'ensemble de la section transversale du conduit de passage (3).

7. Pompe péristaltique selon l'une quelconque des revendications 1 à 6,
**caractérisée en ce que** le moyen (8) susceptible d'être insufflé et aspiré est relié à un insufflateur, respectivement aspirateur (14), susceptible d'insuffler ou d'aspirer dans le moyen (8) en quelques dixièmes de secondes un fluide, de préférence un gaz, tel que l'hélium.

8. Pompe péristaltique selon la revendication 7,
**caractérisée en ce que** l'insufflateur ou l'aspirateur (14) peut fonctionner en étant commandé ou régulé par une valeur de mesure ou de régulation.

9. Pompe péristaltique selon la revendication 8,
**caractérisée en ce que** la valeur de mesure est une valeur de tension artérielle, de circulation sanguine ou une valeur d'ECG.

10. Pompe péristaltique selon l'une quelconque des revendications 1 à 9,
**caractérisée en ce que** dans le corps de passage, il est prévu un moyen pour éliminer par filtration au moins un composant du sang, de préférence pour la filtration de lipides sanguins.

11. Pompe péristaltique selon l'une quelconque des revendications 1 à 10,
**caractérisé en ce que** le moyen (8) susceptible d'être insufflé ou aspiré est relié à un insufflateur ou à un aspirateur(14), susceptible d'insuffler et d'aspirer un fluide, de préférence un gaz, comme de l'hélium de façon rythmée dans le moyen (8), de sorte qu'un écoulement de fluide (F) soit induit le long du conduit de passage (3) avec une surpression extrêmement faible, et qu'il se forme à l'intérieur du corps de passage de préférence un écoulement de fluide axial laminaire, c'est-à-dire sans augmentation d'ondes de pression.

12. Pompe péristaltique selon l'une quelconque des revendications 1 à 11,
**caractérisée en ce que** le moyen (8) susceptible d'être insufflé ou aspiré est relié à un dispositif de pompe introduit dans le corps, susceptible d'insuffler et de d'aspirer au moins partiellement un fluide, de préférence un gaz, comme de l'hélium de façon rythmée dans le moyen, (8) de sorte qu'un écoulement de fluide (F) soit induit le long du conduit de passage (3) avec une surpression extrêmement faible, et qu'il se forme à l'intérieur du corps de passage de préférence un écoulement de fluide axial laminaire, c'est-à-dire sans augmentation d'ondes de pression, et **en ce que** le dispositif de pompe prévoit un système d'actionneurs de la pompe susceptible d'être entraîné par le mouvement respiratoire de la cage thoracique et/ou par la diaphragme.

13. Pompe péristaltique selon la revendication 12,
**caractérisée en ce que** le système d'actionneurs de la pompe prévoit un volume de réservoir relié avec la cage thoracique et/ou avec le diaphragme, qui est entouré d'une enveloppe élastique et qui est déformable par le mouvement respiratoire, et **en ce qu'**à l'intérieur du volume de réservoir, il est prévu au moins une partie du fluide, qui est chassée initialement par le mouvement respiratoire hors du volume de réservoir, pour l'insufflation dans le moyen (8) ou qui est aspiré dans le volume de réservoir, pour l'aspiration hors du moyen (8).
